# EUROPÄISCHE PATENTSCHRIFT

(11) **EP 2 895 246 B1**
(45) Veröffentlichungstag und Bekanntmachung des Hinweises auf die Patenterteilung: **13.12.2017**
(21) Anmeldenummer: 13774603.8
(22) Anmeldetag: 17.09.2013
(51) Int. Cl.: B01D 15/08

(54) **ABTRENNUNG VON PROTEINEN**
SEPARATION OF PROTEINS
SÉPARATION DE PROTÉINES

(30) Priorität: 17.09.2012 DE 102012018234
(43) Veröffentlichungstag der Anmeldung: 22.07.2015
(73) Patentinhaber: MagnaMedics GmbH, 52068 Aachen (DE)
(72) Erfinder: RUSU, Viorel, 6471 KN Eygelshoven (NL); GÖTHEL, Sven, 53844 Troisdorf (DE)
(74) Vertreter: Sparing, Rolf Klaus
(86) Internationale Anmeldenummer: PCT/EP2013/002795
(87) Internationale Veröffentlichungsnummer: WO 2014/040754

(56) Entgegenhaltungen:
- YAN LI ET AL: "Cerium Ion-Chelated Magnetic Silica Microspheres for Enrichment and Direct Determination of Phosphopeptides by Matrix-Assisted Laser Desorption Ionization Mass Spectrometry", JOURNAL OF PROTEOME RESEARCH, Bd. 7, Nr. 4, 29. Februar 2008 (2008-02-29), Seiten 1767-1777, XP055095787, ISSN: 1535-3893, DOI: 10.1021/pr070385l
- X. XU ET AL: "Synthesis of Magnetic Microspheres with Immobilized Metal Ions for Enrichment and Direct Determination of Phosphopeptides by Matrix-Assisted Laser Desorption Ionization Mass Spectrometry", ADVANCED MATERIALS, Bd. 18, Nr. 24, 18. Dezember 2006 (2006-12-18), Seiten 3289-3293, XP055095851, ISSN: 0935-9648, DOI: 10.1002/adma.200601546
- SAITO K ET AL: "Determination of anabolic steroids in human urine by automated in-tube solid-phase microextraction coupled with liquid chromatographymass spectrometry", JOURNAL OF PHARMACEUTICAL AND BIOMEDICAL ANALYSIS, NEW YORK, NY, US, vol. 52, no. 5, 5 September 2010 (2010-09-05), pages 727-733, XP027030287, ISSN: 0731-7085 [retrieved on 2010-02-25]
- THOMAS ANDREAS ET AL: "Determination of prohibited, small peptides in urine for sports drug testing by means of nano-liquid chromatography/benchtop quadrupole orbitrap tandem-mass spectrometry", JOURNAL OF CHROMATOGRAPHY, vol. 1259, 28 June 2012 (2012-06-28), pages 251-257, XP028939444, ISSN: 0021-9673, DOI: 10.1016/J.CHROMA.2012.07.022

## Beschreibung

Die Erfindung betrifft ein Verfahren zur selektiven Abtrennung von Proteinen aus flüssigen biologischen Materialien, die bezogen auf die Gesamtmenge einen Anteil von weniger als 10 Gew. % einer Spurenkomponente, die ausgewählt sind aus einer Gruppe von Verbindungen wie entzündungshemmenden Immunosuppressiva, Antiarrythmika, Nicht-Protein-Biomarker, Drogen, Dopingmittel, Mycotoxinen, Antidepressiva, Antiepileptika, Antipsychotika, Antibiotika enthalten

Biologische Materialien enthalten häufig einen hohen Gehalt an Proteinen neben einem geringen Gehalt an weiteren Komponenten. Diese Komponenten lassen sich bei dem hohen Proteingehalt nur schwer qualitativ, analytisch nachweisen, z.B. bei Kontrolle des Medikamentenspiegels bei der Gabe von immunosuppressiven Medikamenten oder deren Metaboliten aus Blut, Plasma oder Serumproben.

Flüssigen biologischen Materialien wie Plasmaproben (und andere klinisch relevante Flüssigkeiten) werden typischerweise mittels ELISA-assays (Linked Immunosorbent Assay) auf den quantitativen Inhalt von Biomarkern (niedermolekularen, organischen Verbindungen) hin untersucht, um einen Krankheitsverlauf aufzuzeichnen bzw. eine Krankheit überhaupt erst zu diagnostizieren. In den letzten Jahren beginnt sich die LC-MS (bzw. LC-MS/MS) als alternative zu ELISA-assays zu etablieren. Typischerweise wird hierzu die Plasmaprobe mittels Präzipitation und anschließender Zentrifugation von Plasmaproteinen abgetrennt und anschließend die niedermolekularen Verbindungen in einer Reversed-Phase-LC aufgetrennt, quantifiziert (Fläche des LC-Diagramms) und die Molmasse per Massenspektrometrie (MS) analysiert.

Die Proteinabtrennung wird dabei üblicherweise manuell durchgeführt, da Zentrifugationsschritte (oder Alternativen wie Absaugen durch eine Membran mittels Vakuum oder Druck) schwierig zu automatisieren sind.

Es ist bekannt, dass abhängig vom Ursprung der Probe und der analytischen Fragestellung, der qualitative wie auch quantitative Anteil einer Spurenkomponente aus einer biologischen Probe auf unterschiedlichen instrumentellen Plattformen und für unterschiedliche Endanwendungen, wie klinische Kontrollen, Dopinguntersuchungen, Forensische und Toxikologische Gutachten und Untersuchungen, durchgeführt werden können. Unabhängig von der technologischen Entwicklung bei der Auslesung der experimentellen Daten, wie z.B. der technologischen Entwicklung in der Massenspektrometrie, ist nach wie vor eine Vorbehandlung der analytischen Probe zwecks Komplexitätsreduktion notwendig.

Im Wesentlichen ist der analytische Prozess von der Probenentnahme bis zum Endergebnis in fünf Schritte unterteilt:
1) Probenentnahme,
2) Probenvorbereitung,
3) Probenfräktionierung,
4) Analytdetektion und
5) Datenauswertung.

In den meisten Fällen wird die wesentliche Zeit auf die Schritte 1) und 2) verwendet.

So ist im Journal of Proteome Research Band 7, Nr. 4 Seiten 1767-1777 wird ein Verfahren offenbart, indem chelatisierte Ce⁴⁺ Silica-Partikel zur Abtrennung von Phosphopeptiden dienen. Das Verfahren dient dabei zur direkten Abtrennung und Analyse der Phosphopeptide.

Ferner ist in Advanced Materials, Band 18, Nr. 24 vom 18. Dezember 2006, Seiten 3289-3293 ein Verfahren zur Herstellung magnetischer Metallverbindungen zur Anreichung und Bestimmung von Phosphopeptiden beschrieben, wobei die zu erhaltenen Peptide nachfolgenden spektroskopischen Untersuchungen zugeführt werden sollen.

Weiterhin ist aus dem Journal of Pharmaceutical and Biomedical Analysis, Band 52, Nr.5, vom 05. September 2010, Seiten 727 bis 733 bekannt, dass anabole Steroide aus Urinproben durch Festphasenextraktion in Verbindung mit LC/MS isoliert und analysiert werden können.

Auch ist im Journal of Chromatography, Band 1259, Seiten 251-257 offenbart, dass Peptide mit einem Gewicht von weniger als 1,5 kD isoliert und einer Massenspektroskopischen Untersuchung zugeführt werden können.

Aufgabe der vorliegenden Erfindung eine Abtrennung von Proteinen zu erzielen, um die im Überstand verbliebenen Spurenkomponenten selektiv einer geeigneten, sauberen Analyse unterziehen zu können (LC/MS etc.).

Die Aufgabe wird durch das beanspruchte Verfahren gelöst. Dieses Verfahren enthält u.A. die Schritte:
a) Zugeben mindestens eines polaren, organischen Lösungsmittels zu dem flüssigen biologischen Material, wobei das Lösungsmittel ein Dipolmoment im Bereich von 1,6 bis 4,0 Debye und
b) Zugeben von magnetischen Kieselgel-Partikeln zum flüssigen biologischen Material zur Adsorption der Proteine,
c) magnetisches Entfernen der Kieselgel-Partikel mit den adsorbierten Proteinen aus dem flüssigen biologischen Material und Erhalt eines flüssigen Rückstands, wobei die Spurenkomponenten selektiv in der Flüssigkeit verbleiben und nachfolgend analysiert werden.

Eine bevorzugte Ausführungsform des erfindungsgemäßen Verfahrens ist dadurch gekennzeichnet, dass die Kieselgel-Partikel gegebenenfalls einen oder mehrere magnetische Kerne besitzen und einen Durchmesser im Bereich von 20 nm bis 500 µm aufweisen. Bevorzugt enthalten die Kieselgel-Partikel 0 bis 30 magnetische Kerne.

Die Abtrennung der Kieselgel-Partikel mit den adsorbierten Proteinen kann in einem Magnetfeld, oder durch Adsorption der Proteine an nicht-magnetische Kieselgel-Partikel und Abtrennung der Kieselgel-Partikel mittels Druck, Zentrifugal- bzw. Gravitationskraft erfolgen.

Es wurde daher ein bevorzugtes Verfahren zur selektiven Abtrennung von Proteinen aus flüssigen biologischen Materialien gefunden, die bezogen auf die Gesamtmenge einen kleinen Anteil von Spurenkomponenten enthalten, durch Addition von
a) polaren, organischen Lösungsmitteln mit einem Dipolmoment im Bereich von 1,6 bis 4,0 Debye und
b) Kieselgel-Partikeln mit einem oder mehreren magnetischen Kernen, Adsorption der Proteine an die magnetischen Kieselgel-Partikel und Abtrennung der magnetischen Kieselgel-Partikel mit den adsorbierten Proteinen in einem Magnetfeld, oder Adsorption der Proteine an nicht-magnetische Kieselgel-Partikel und Abtrennung der Kieselgel-Partikel mittels Druck, Zentrifugal bzw. Gravitationskraft, wobei die Spurenkomponenten in der Flüssigkeit verbleiben.

Flüssige biologische Materialien im Rahmen der vorliegenden Erfindung sind vorzugsweise wässrige Körperflüssigkeiten von Mensch oder Tier.

Flüssige biologische Materialien im Rahmen der vorliegenden Erfindung können beispielsweise Plasma, Serum, Speichel, Tränen, Gehirnflüssigkeit, Gewebeflüssigkeit, Fruchtwasser, Follikelflüssigkeit, Vollblut oder hämolysiertes Blut, Urin, Liquor, wie Liquor cerebrospinalis, insterstitielle Flüssigkeiten aber auch z.B. Fermentationsmedien sein.

Die flüssigen biologischen Materialien sind in der Regel wässrige Lösungen, die außer Proteinen Salze und weitere organische Komponenten enthalten.

Im Rahmen der vorliegenden Erfindung enthalten die flüssigen biologischen Materialien bezogen auf die Gesamtmenge einen kleinen Anteil weiterer organischer Komponenten ("Spurenkomponenten"). Der Anteil der Spurenkomponenten in den flüssigen biologischen Materialien ist bezogen auf die Gesamtmenge kleiner als 10 Gew. %. Im Rahmen der Erfindung wird ein Anteil der Spurenkomponente im Bereich von 10exp-12 bis 5 Gew. % bevorzugt. Im Besonderen wird ein Anteil der Spurenkomponente im Bereich von 10 exp-9 bis 2 Gew. % bevorzugt.

Nach dem erfindungsgemäßen Verfahren lassen sich überraschenderweise die Proteine aus den biologischen Flüssigkeiten leicht und schnell abtrennen, ohne dass die Zusammensetzung und die Mengen der Spurenkomponenten verändert werden.

Spurenkomponenten im Rahmen der vorliegenden Erfindung sind:
a) entzündungshemmende Immunsuppressiva, wie beispielsweise Azathioprin, Mercaptopurin, Mycophenolat, Mofetil, Mycophenolsäure, Sirolimus (Rapamycin), Leflunomid, Teriflunomid, Methotrexat, Tacrolimus, Ciclosporin, Pimecrolimus, Gusperimus, Lenalidomid, etc.
b) Antiarrythmika, wie beispielsweise Procainamide, Quinidine , Disopyramide A, Lidocaine, Phenytoin, Mexiletine, Tocainide, Flecainide, Propafenone, Moricizine, Lidocaine, Phenytoin, Mexiletine, Propanolol, Esmolol, Timolol, Metoprolol, Atenolol, Bisoprolol, Sotalol, Ibutilide, Dofetilide, Droneradone, E-4031, Verapamil, Diltiazem, Adenosine, Digoxin, etc.
c) Nicht-Protein Biomarker, wie beispielsweise Östrogene und Sexualhormone, Ascorbinsäure, Carotinoide, Zytokine, etc.
d) Drogen, wie beispielsweise Heroin, Kokain, Amphetamin, Morphin, etc.
e) Dopingmittel, wie beispielsweise
e1) Anabole Wirkstoffe wie anabol-androgene Steroide (AAS), zum Beispiel 1-Androstendiol, 1-Androstendion, Bolandiol, Bolasterone, Boldenon, Boldione, Calusterone, Clostebol, Danazol, Dehydrochlormethyltestosterone, Desoxymethyltestosterone, Drostanolone, Ethylestrenol Fluoxymesteron, Formebolone, Furazabol, Androstendiol, Androstendion, Dihydrotestosteron, Testosteron, Clenbuterol, Tibolon, Zeranol, Zilpaterol.
e2) Beta-2 Agonisten, wie Abediterol, Amibegron, Arbutamin, Arformoterol, Bambuterol, Bitolterol, Carbuterol, Clenbuterol, Fenoterol, Formoterol, Hexoprenalin, Indacaterol, Isoetarine, Isoprenalin, Levosalbutamol, Olodaterol, Pirbuterol, Procaterol, Adrenalin, Ractopamine, Reproterol, Rimiterol, Salbutamol, Salmeterol, Solabegron, Terbutalin, Tulubuterol.
e3) Hormon-Antagonisten und-Modulatoren wie Anastrozol, Androstatrienedione, Exemestan, Formestan, Letrozol, Testolacton, Raloxifen, Tamoxifen, Toremifen, Clomiphen, Cyclofenil, Fulvestrant.
e4) Diuretika wie Acetazolamid, Amilorid, Bumetanid, Canrenon, Chlorthalidon, Etacrynsäure, Furosemid, Indapamid, Metolazon, Spironolacton, Thiazide, Triamteren.
e5) Stimulanzien wie Adrafinil, Amfepramon, Amiphenazol, Amphetamin, Amphetaminil, Benfluorex, Benzphetamin, Benzylpiperazin, Bromantan, Clobenzorex, Kokain, Cropropamide, Crotetamide, Dimethylamphetamine, Etilamphetamine, Famprofazone, Fencamine, Fenetyllin, Fenfluramin, Fenproporex, Furfenorex, Mefenorex, Mephentermin, Mesocarb, Methamphetamin, (d-), p-Methylamphetamin, Methylendioxyamphetamin, Methylendioxymethamphetamin, Methylhexanamin, Modafinil, Norfenfluramine, Phendimetrazin, Phenmetrazin, Phentermine, 4-phenylpiracetam, Prenylamin, Prolintan.
e6) Betäubungsmittel wie Buprenorphin, Dextromoramid, Diamorphin (Heroin), Fentanyl und seine Derivate, Hydromorphon, Methadon, Morphin, Oxycodon, Oxymorphon, Pentazocin, Pethidin.
f) Mycotoxinen, wie beispielsweise Aflatoxin B1, Fumonisin B1 und B2, Ochratoxin A, Patulin und Zearalenon.
g) Antidepressiva, wie beispielsweise Celexa, Cipramil, Lexapro, Cipralex, Seroplex, Lexamil, Prozac, Sarafem, Symbyax, Luvox, Paxil, Aropax, Zoloft, Viibryd, Pristiq, Cymbalta, Ixel, Effexor, Tolvon, Remeron, Avanza, Zispin, Strattera, Mazanor, Sanorex, Edronax, Vivalan, Wellbutrin, Zyban, Stabion, Coaxil, Tatinol, Amineptine, Valdoxan, Melitor, Thymanax, Elavil, Endep, Anafranil, Adapin, Sinequan, Tofranil, Surmontil, Norpramin, Pamelor, Aventyl, Noritren, Vivactil, Marplan, Aurorix, Manerix, Nardil, Eldepryl, Emsam, Parnate, Nicotine.
h) Antiepileptika, wie beispielsweise Acetazolamid,Carbamazepin, Clobazam, Clonazepam, Diazepam, Ethosuximid, Felbamat, Gabapentin, Lacosamid, Lamotrigin, Levetiracetam, Mesuximid, Midazolam, Oxcarbazepin, Phenobarbital, Phenytoin, Pregabalin, Primidon, Rufinamid, Stiripentol, Sultiam, Tiagabin, Topiramat, Valproinsäure.
i) Antipsychotika, wie beispielsweise Aripiprazol, Olanzapin, Paliperidon, Quetiapin, Risperidon, Ziprasidon, Solian, Abilify, Leponex und/oder
j) Antibiotika, wie beispielsweise Ciprofloxacin, Fosfomycin, Fusafungin, Rifaximin, Telithromycin, Vancomycin, Cephalosporine, Makrolid-Antibiotika, Penicilline, Sulfonamide und Trimethoprim, Tetrazykline, Ethambutol, Isoniazid, Myambutol, Pyrazinamid, Rifampicin, Streptomycin, Imipenem, Cilastin, Meropenem, Lincosamide, Monobactam.

Besonders bevorzugte Spurenkomponenten für das erfindungsgemäße Verfahren sind Immunosuppressiva, Antiarythmika, Nicht-Protein-Biomarker, Dopingmittel, Antidepressiva und Antibiotika.

Proteine, die im Rahmen der vorliegenden Erfindung abgetrennt werden können, können beispielsweise Serumalbumine, Immunglobuline, Fibrinogen, Regulator Proteine sein.

Im Rahmen der vorliegenden Erfindung können die flüssigen biologischen Materialien mehr als 0,1 Gew. % Proteine enthalten.

Im Rahmen der vorliegenden Erfindung ist ein Proteingehalt im Bereich von 0,01 bis 25 Gew. % bevorzugt, insbesondere bevorzugt im Bereich von 0,1 bis 12 Gew. %.

Die Spurenkomponenten im Rahmen der vorliegenden Erfindung sind im Allgemeinen in dem polaren, organischen Lösungsmitteln gut löslich.

Im Allgemeinen weisen die Spurenkomponenten in dem polaren, organischen Lösungsmitteln eine Löslichkeit von mindestens 0,1 pg/l, bevorzugt im Bereich von 1 ng/l bis 500 mg/l, auf.

Polare, organische Lösungsmittel im Rahmen der vorliegenden Erfindung haben im Allgemeinen ein Dipolmoment im Bereich von 1,6 bis 4,0 Debye, bevorzugt von 1,69 bis 3,96 Debye.

Als polare, organische Lösungsmittel seien beispielsweise genannt: Aceton, Acetonitril, Ethanol, Methanol, Propanol, iso-Propanol, Dimethylsulfoxid, Polyethylenglykol-(PEG).

Bevorzugte polare, organische Lösungsmittel sind: Aceton, Acetonitril, Ethanol und iso-Propanol.

Die polaren, organischen Lösungsmittel können einzeln oder im Gemisch verwendet werden.

Die Mischungen werden beispielsweise so eingestellt, dass Alkohole wie Ethanol und iso-Propanol in verschiedenen Gewichtsanteilen oder Acetonitril zusammen mit einem Alkohol wie z.B. iso-Propanol oder Ethanol oder Kombinationen aus Alkoholen wie Ethanol und iso-Propanol mit verschieden Gewichtsanteilen mit z.B. Acetonitril kombiniert werden.

Das erfindungsgemäße Verfahren wird bevorzugt an einem Adsorbens aus Kieselgel-Partikeln mit einem oder mehreren magnetischen Kernen und mit einer Porengröße im Bereich von 2 bis 50 nm, bevorzugt 5 bis 30 nm und einer inneren Oberfläche im Bereich von 0,1 bis 400 m²/g ; bevorzugt 10 bis 200 m²/g, durchgeführt. Die Trennung der Proteine, adsorbiert an die Kieselgel-Partikel-Festphase, von den Spurenkomponenten erfolgt dabei durch Anlegen eines magnetischen Feldes.

Alternativ können die Proteine, nach Zugabe eines organischen, polaren Lösungsmittelgemisches, wie oben beschrieben, an nicht-magnetische Kieselgel-Partikel adsorbiert werden. Die Trennung der gebundenen Proteine von den in Lösung verbliebenen Spurenkomponenten kann dabei mittels Druck, Gravitations- oder Zentrifugalkraft erfolgen.

Die Kieselgel-Partikel können dabei als isolierte sphärische Partikel, als monolithische Kieselgel-Phasen oder als Membranen vorliegen.

Insbesondere bei monolithischen Kieselgel-Phasen als auch bei Kieselgelbasierten Membranen wird durch Aufbau einer Druckdifferenz die Flüssigkeit mit den gelösten Spurenkomponenten gemäß der Druckdifferenz durch das Kieselgel-Material geleitet und von den adsorbierten, zurückgehaltenen Proteinen getrennt. Die Druckdifferenz wird dabei mittels Überdruck/Unterdruck an der Membran/monolithischen Phase der Membran bzw. monolithischen Phase aufgebaut.

Die Partikel haben im Allgemeinen einen Durchmesser im Bereich von 20 nm bis 500 µm, bevorzugt von 200 nm bis 10 µm, insbesondere bevorzugt von 500 nm bis 1,3 µm.

Das Adsorbens aus Kieselgel-Partikeln mit einem magnetischen Kern kann durch Beschichten von Eisenoxid enthaltenden Partikeln mit Kieselgel hergestellt werden.

Die Beschichtung von Eisenoxid enthaltenden Partikeln mit Kieselgel ist an sich bekannt (J.Colloid Interface Sci. 1968, 26, 62 bis 69; Langmuir 2005, 21, 10763 bis 10769; J. Colloid Interface Sci 2005, 283, 392 bis 396).

Die Beschichtung von Eisenoxid enthaltenden Partikeln mit Kieselgel für das erfindungsgemäße Verfahren kann beispielsweise wie folgt durchgeführt werden:
Eine Suspension von Eisenoxid enthaltenden Partikeln in einem Alkohol (z.B. Isopropanol) wird unter starkem Rühren in Gegenwart von Ammoniak zur Beschichtung mit Tetra-ethyl-orthosilicat (TEOS) versetzt. Die Dicke der Beschichtung kann durch die Menge des zugegebenen Tetra-ethyl-orthosilicats gesteuert werden.

Die beschichteten Eisenoxid enthaltenden Partikel werden mit einem Alkohol (z.B Methanol) gewaschen und in Wasser gelagert.

Für das erfindungsgemäße Verfahren werden besonders Kieselgel-Partikel bevorzugt, die aus einer mesoporösen Schicht bestehen, die auf den magnetischen Kern aufgetragen ist und eine Schichtdicke im Bereich von 10 bis 100 nm aufweist.

Magnetische Kerne für die erfindungsgemäßen Kieselgel-Partikel können an sich bekannte Partikel aus Eisenoxid (Fe₂O₃) und Siliciumdioxid, Polystyrol und/oder Polyvinylalkohol sein.

Für das erfindungsgemäße Verfahren werden bevorzugt Eisenoxid-Partikel mit einer mesoporösen Kieselgel-Beschichtung eingesetzt, wie sie in Gegenwart von Polyethylenglykol als porogenes Mittel entsteht.

Im Besonderen bevorzugt werden Kieselgel-Partikel, die aus einer mesoporösen Schicht bestehen, die auf den magnetischen Kern aufgetragen ist und eine Schichtdicke im Bereich von 10 bis 100 nm aufweist, wobei die magnetischen Kerne Maghemit und/oder Magnetit im Bereich von 30 bis 95 Gew% enthalten und einen mittleren Durchmesser im Bereich von 10 nm bis 500 µm aufweisen, wobei die Kieselgel Partikel einen mittleren Durchmesser im Bereich von 20 nm bis 500 µm, bevorzugt 200 nm bis 10 µm, insbesondere bevorzugt 500 nm bis 1,5 µm aufweisen.

Eine bevorzugte Ausführungsform des erfindungsgemäßen Verfahrens ist dadurch gekennzeichnet, dass zu einem Gewichtsteil flüssige biologische Materialien 1.5 bis 4 Gewichtsteile, bevorzugt 2 bis 3 Gewichtsteile, des polaren, organischen Lösungsmittels und 0,02 bis 0,50 Gewichtsteile, bevorzugt 0,05 bis 0,40 Gewichtsteile, der Kieselgel Partikeln Kern gegeben werden.

Die Abtrennung der Kieselgel-Partikel mit einem magnetischen Kern kann mit Hilfe eines magnetischen Separators erfolgen.

Nach der erfindungsgemäßen selektiven Abtrennung von Proteinen aus flüssigen biologischen Materialien enthält die zurückbleibende Flüssigkeit nur wenig Proteine, die weitere Untersuchungen oder eine Isolation der Spurenkomponenten nicht behindern. Der Anteil der Proteine in der zurückbleibenden Flüssigkeit ist im Allgemeinen geringer als 0,0001 Gew. %.

Das erfindungsgemäße Verfahren kann beispielsweise wie in den folgenden Schritten durchgeführt werden:
(i) Bereitstellen von flüssigen biologischen Materialien, die eine oder mehrere Spurenkomponeneten enthalten,
(ii) Kontaktieren der flüssigen biologischen Materialien mit Kieselgel-Partikeln,
(iii) Abtrennung des Proteins durch Zugabe von organischen Lösungsmitteln im vordefinierten Verhältnis
(iv) Verwirbeln und Inkubieren des Gemisches, wobei die Proteine auf der Oberfläche der Partikel adsorbiert werden,
(v) Abtrennen des Überstandes, der die Spurenkomponenten enthält, von den Partikeln, die die adsorbierten Proteine enthalten, durch Anlegen eines magnetischen Feldes bzw. durch Druck, Zentrifugal - oder Gravitationskräfte,
(vi) Abnahme des Überstands mit einer oder mehreren Verbindungen wodurch eine selektive Extraktion von Verbindungen aus der biologischen Probe erfolgt,
(vii) ein weiterer optionaler Schritt, der das Trocknen der Überstandes durch Verdampfen der organischen Lösungsmittelmischung bei erhöhter Temperatur (50 - 85 °C) beinhaltet und anschließend die Probe wieder in einem geringen, definierten Volumen organischen Lösungsmittels aufgenommen wird,
(ix) Analysieren von ein oder mehreren Verbindungen aus (vi) bzw. (vii) mittels Massenspektrometrie, wobei optional eine weitere chromatographische Trennung der Probe erfolgen kann und/oder Immunassays, die monoplex oder multiplex aufgebaut sein können, wobei ein multiplexes Immunassay durch mehrfarbige Partikelkodierung erfolgen kann.

Gegenstand der vorliegenden Offenbarung sind auch biologische Materialien mit geringem Gehalt an Proteinen und Spurenkomponenten die dadurch gekennzeichnet sind, dass Proteinen aus flüssigen biologischen Materialien, die bezogen auf die Gesamtmenge einen kleinen Anteil einer Spurenkomponenten enthalten, durch Addition von
a) polaren, organischen Lösungsmitteln mit einem Dipolmoment im Bereich von 1,6 bis 4,0 Debye und
b) Kieselgel-Partikeln, durch Adsorption der Proteine an die Kieselgel-Partikel abgetrennt werden und nach Abtrennung der Kieselgel-Partikel mit den adsorbierten Proteinen, die Spurenkomponenten in der Flüssigkeit verbleiben.

In einer bevorzugten Ausführungsform der vorliegenden Erfindung sind die biologischen Materialien mit geringem Gehalt an Proteinen und aktiven Komponenten beispielsweise aus einer der Gruppen
a) der entzündungshemmenden Immunosuppressiva,
b) der Antiarrythmika,
c) der Nicht-Protein-Biomarker,
d) der Drogen
e) der Dopingmittel
f) der Mycotoxinen
g) der Antidepressiva
h) der Antiepileptika
i) der Antipsychotika
j) der Antibiotika
ausgewählt.

Gegenstand der vorliegenden Offenbarung ist auch die Verwendung von biologischen Materialien mit geringem Gehalt an Proteinen und Spurenkomponenten, beispielsweise in analytischen oder präparativen Untersuchungen.

Insbesondere in der Analytik von Spurenkomponenten neben Proteinen als Hauptkomponente eröffnet die vorliegende Erfindung vorteilhafterweise eine einfache Durchführung mit geringen Fehlern, die besonders durch die Komplexität des Systems bedingt sein können.

### Beispiele:

### Beispiel 1

### Eisenoxid-Partikel mit einer mesoporösen Kieselgel Beschichtung in Gegenwart von Polyethylenglykol

10 ml Eisenoxid-Partikel (e.g. MagSi-S beads der Firma MagnaMedics) mit einer Konzentration von 20 mg/ml werden in einem Magnetfeld abgetrennt und dann in 30 ml Polyethylenglycol (mittleres Molekulargewicht 400) 10 ml Isopropanol und 2 ml Wasser unter starkem Rühren dispergiert. Zu dieser Mischung gibt man 2 ml einer 25 Gew % Ammoniaklösung und 0,75 ml Tetra-ethyl-orthosilicat (TEOS). Die Beschichtung erfolgt innerhalb von 6 Stunden unter gleichmäßigem Rühren.

Die beschichteten Eisenoxid enthaltenden Partikel werden mit 40 ml Wasser gewaschen. Die Endkonzentration an säuregruppenfreien Kieselgel-Partikeln beträgt 20 mg/ml.

### Beispiel 2

Die Probenvorbereitung für die Bestimmung von Cortisol lyophilisiertem Humanserum

### Materialien:

- Lyophilisiertes Humanserum mit Cortisol,
- Magnetic Beads 300 mg/ml in Acetonitril nach Beispiel 1
- Organische Lösungsmittel-Mix, 100% Acetonitril
- Mikrotiterplatten, UV-Typ mit flachem Boden
- Mikroplatten-Reader;
   1. Humanes, lyophilisiertes Serum wird in 1,5 ml sterilem Wasser wieder aufgenommen, so dass die Endkonzentration an Kortisol 0,277 µg/ml beträgt.
   2. 30, 40, 60 und 80 µl aufgenommenes Serum wird in Mikrotiterplatten überführt (UV durchlässiges Format).
   3. Zu dem Serum werden 25 µl MagnaMedics-Partikel-Lösung und anschließend die organische Lösungsmittelmischung zugegeben. Das Volumen des organischen Lösungsmittelgemisches beträgt dabei das 2-fache des Serum Volumens.
   4. Anschließend wird die Lösung gründlich gemischt und der Reaktionsansatz 2 min. bei Raumtemperatur inkubiert.
   5. Vor der Magnetkollektion wird die Probe 5 s. mit Ultraschall behandelt.
   6. Die Magnetpartikel werden in einem magnetischen Separator vollständig gesammelt.
   7. Der Überstand wird abgenommen und bei 242 nm im Mikrotiterplattenformat vermessen.

### Ergebnisse:

Als Negativkontrolle wird eine Serumprobe, die kein Kortisol enthält, verwendet, um den Hintergrund zu bestimmen. Die gemessene optische Dichte (OD) korreliert dabei direkt mit der Kortisolkonzentration.

| | | | | | |
|---|---|---|---|---|---|
| Serum mit additiv zugeführtem Kortisol | 30 | 40 | 60 | 80 | Serum ohne additiv zugeführtes Kortisol |
| Steriles Wasser (µl) | 70 | 60 | 40 | 20 | 0 |
| Magnetische Partikel (µl) | 25 | 25 | 25 | 25 | 25 |
| Acetonitril (µl) | 200 | 200 | 200 | 200 | 200 |
| Transfervolumen(µl) | 200 | 200 | 200 | 200 | 200 |
| OD bei 242 nm | 0.3554 | 0.4229 | 0.6590 | 0.8571 | 0.0948 |
| | 0.3371 | 0.3670 | 0.5410 | 0.6841 | 0.0687 |
| Durchschnittliche OD | 0.3462 | 0.3949 | 0.6000 | 0.7706 | 0.8175 |
| Korrigierte OD | 0.2644 | 0.3131 | 0.5182 | 0.6888 | - |
| Menge an gemessenem Kortisol (µg) | 8,31 | 11,08 | 16,62 | 22,16 | - |

## Patentansprüche

1. Verfahren zur selektiven Abtrennung von Proteinen aus flüssigen biologischen Materialien,
die bezogen auf die Gesamtmenge einen Anteil von weniger als 10 Gew. % einer Spurenkomponente, die ausgewählt sind aus einer Gruppe von Verbindungen wie entzündungshemmenden Immunosuppressiva, Antiarrythmika, Nicht-Protein-Biomarker, Drogen, Dopingmittel, Mycotoxinen, Antidepressiva, Antiepileptika, Antipsychotika, Antibiotika enthalten, **gekennzeichnet durch** die Schritte
a) Zugeben mindestens eines polaren, organischen Lösungsmittels zu dem flüssigen biologischen Material, wobei das Lösungsmittel ein Dipolmoment im Bereich von 1,6 bis 4,0 Debye und
b) Zugeben von magnetischen Kieselgel-Partikeln zum flüssigen biologischen Material zur Adsorption der Proteine,
c) magnetisches Entfernen der Kieselgel-Partikel mit den adsorbierten Proteinen aus dem flüssigen biologischen Material und Erhalt eines flüssigen Rückstands, wobei die Spurenkomponenten in der Flüssigkeit verbleiben und nachfolgend analysiert werden.

2. Verfahren nach Anspruch 1, **dadurch gekennzeichnet, dass** die Kieselgel-Partikel zumindest einen oder mehrere magnetische Kerne besitzen.

3. Verfahren nach Anspruch 1 und 2, **dadurch gekennzeichnet, dass** die Kieselgel-Partikel einen Durchmesser im Bereich von 20 nm bis 500 µm aufweisen.

4. Verfahren nach einem der Ansprüche 1 bis 3, **dadurch gekennzeichnet, dass** das flüssige biologische Material wässrige Körperflüssigkeiten von Mensch oder Tier sind.

5. Verfahren nach Anspruch 1 oder 4, **dadurch gekennzeichnet, dass** das flüssige biologische Material Plasma, Serum, Speichel, Tränen, Hirnflüssigkeit, Gewebeflüssigkeit, Fruchtwasser, Follikelflüssigkeit, Vollblut, hämolysiertes Blut, Urin, Zerebrospinalflüssigkeit umfasst.

6. Verfahren nach einem der Ansprüche 1 bis 5, **dadurch gekennzeichnet, dass** die Spurenkomponenten in dem mindestens einen polaren, organischen Lösungsmittel löslich sind.

7. Verfahren nach einem der Ansprüche 1 bis 6, **dadurch gekennzeichnet, dass** die Konzentration der Spurenkomponenten eine Mindestkonzentrationswert von 100pg/l aufweisen.

8. Verfahren nach einem der Ansprüche 1 bis 7, **dadurch gekennzeichnet, dass** die Zugabe mindestens eines polaren, organischen Lösungsmittels zu dem flüssigen biologischen Material das 1,5- bis 4-fache der Gewichtsteile des mindestens einen polaren, organischen Lösungsmittels beträgt.

9. Verfahren nach einem der Ansprüche 1 bis 8, **dadurch gekennzeichnet, dass** das Zugeben der Kieselgel-Partikel zum flüssigen biologischen Material das 0,05 bis 0,4-fache an Gew.-Teile der Kieselgel-Partikel zu einem Gewichtsteil des flüssigen biologischen Materials ausmacht.

10. Verfahren nach einem der Ansprüche 1 bis 9, **dadurch gekennzeichnet, dass** das Entfernen der an die Kieselgel-Partikel adsorbierten Proteine aus der die Flüssigkeit durch Druck-, Zentrifugalkraft und/oder einem Magnetfeld erfolgt.

11. Verfahren nach einem der Ansprüche 1 bis 10, **dadurch gekennzeichnet, dass** die Spurenkomponenten Nicht-Protein Biomarker, insbesondere Östrogene, Carotinoide und Zytokine umfassen.

## Claims

1. A method for selectively removing proteins from liquid biological materials
which, based on the total amount, contain a proportion of less than 10 weight % of a trace component, selected from a group of compounds like anti-inflammatory immunosuppressants, antiarrhythmics, nonprotein biomarkers, drugs, doping substances, mycotoxins, antidepressants, antiepileptics, antiprychotics and antibiotics, comprising the steps
a) adding at least one polar, organic solvent to the liquid biological material, the solvent having a dipole moment within the range from 1.6 to 4.0 debye and
b) adding magnetic silica gel particles to the liquid biological material to adsorb the proteins,
c) magnetically removing the magnetic silica gel particles containing the adsorbed proteins from the liquid biological material and receiving of a liquid residue, wherein the trace components remain in the liquid and are subsequently analyzed.

2. The method as claimed in claim 1, **characterized in that** the silica gel particles have at least one or more magnetic cores.

3. The method as claimed in claim 1 and 2, **characterized in that** the silica gel particles have a diameter within the range from 20 nm to 500 µm.

4. The method as claimed in any of the claims 1 to 3, **characterized in that** the liquid biological materials are aqueous human or animal body fluids.

5. The method as claimed in claim 1 or 4, **characterized in that** the liquid biological material comprises plasma, serum, saliva, teardrops, brain fluid, tissue fluid, amniotic fluid, follicular fluids, whole blood, hemolyzed blood, urine, cerebrospinal liquid.

6. The method as claimed in any of the claims 1 to 5, **characterized in that** the trace components are soluble in the at least one polar, organic solvent.

7. The method as claimed in any of the claims 1 to 6, **characterized in that** the trace components have a minimum concentration of 100 pg/l.

8. The method as claimed in any of the claims 1 to 7, **characterized in that** the adding of at least one polar, organic solvent to the liquid biological material encompasses 1.5 to 4 times of the parts per weight of the at least one polar, organic solvent.

9. The method as claimed in any of the claims 1 to 8, **characterized in that** the adding of silica gel particles to the liquid biological material constitutes 0.05 to 0.4 times the silica gel particles to one part by weight of the liquid biological material in parts by weight.

10. The method as claimed in any of the claims 1 to 9, **characterized in that** removing the proteins adsorbed to the silica gel particles from the liquid biological material is achieved by compressive force, centrifugal force and/or a magnetic field.

11. The method as claimed in any of the claims 1 to 10, **characterized in that** the trace components comprise nonprotein biomarkers, particularly estrogens, carotenoids and cytokines.

## Revendications

1. Procédé de séparation sélective de protéines à partir de matières biologiques liquides qui contiennent, sur la base de la quantité totale, une proportion de moins de 10% d'un composant-trace, qui sont choisis dans un groupe de composés tels que les immunosuppresseurs anti-inflammatoires, les anti-arythmiques, les biomarqueurs non protéiques, les médicaments, les agents de dopage, les mycotoxines, les antidépresseurs, les antiépileptiques, les antipsychotiques, les antibiotiques, ledit procédé étant **caractérisé par** les étapes consistant à
a) ajouter au moins un solvant organique polaire à la matière biologique liquide, le solvant ayant un moment dipolaire dans la gamme allant de 1,6 à 4,0 Debye et
b) ajouter des particules de gel de silice magnétiques à la matière biologique liquide pour l'adsorption des protéines,
c) retirer par moyens magnétiques les particules de gel de silice, pourvues des protéines adsorbées, de la matière biologique liquide, et obtenir un résidu liquide, les composants-traces restant dans le liquide et étant analysés ultérieurement.

2. Procédé selon la revendication 1, **caractérisé en ce que** les particules de gel de silice possèdent au moins un ou plusieurs noyaux magnétiques.

3. Procédé selon la revendication 1 et 2, **caractérisé en ce que** les particules de gel de silice ont un diamètre dans la gamme allant de 20 µm à 500 µm.

4. Procédé selon l'une quelconque des revendications 1 à 3, **caractérisé en ce que** la matière biologique liquide est constituée par des fluides corporels aqueux humains ou animaux.

5. Procédé selon la revendication 1 ou 4, **caractérisé en ce que** la matière biologique liquide est du plasma, du sérum, de la salive, des larmes, du liquide céphalorachidien, du liquide tissulaire, du liquide amniotique, du liquide folliculaire, du sang entier, du sang hémolysé, de l'urine, du liquide cérébrospinal.

6. Procédé selon l'une des revendications 1 à 5, **caractérisé en ce que** les composants-traces sont solubles dans l'au moins un solvant organique polaire.

7. Procédé selon l'une des revendications 1 à 6, **caractérisé en ce que** la concentration des composants-traces a un niveau de concentration minimum de 100 pg/l.

8. Procédé selon l'une des revendications 1 à 7, **caractérisé en ce que** l'ajout d'au moins un solvant organique polaire à la matière biologique liquide est de 1,5 à 4 fois la partie en poids de l'au moins un solvant organique polaire.

9. Procédé selon l'une des revendications 1 à 8, **caractérisé en ce que** l'ajout des particules de gel de silice à la matière biologique liquide constitue de 0,05 à 0,4 fois la partie en poids des particules de gel de silice par rapport à une partie en poids de matière biologique liquide.

10. Procédé selon l'une des revendications 1 à 9, **caractérisé en ce que** le retrait des protéines du liquide, adsorbées aux particules de gel de silice, est effectué sous l'action de la pression, de la force centrifuge et/ou d'un champ magnétique.

11. Procédé selon l'une des revendications 1 à 10, **caractérisé en ce que** les composants-traces comprennent des biomarqueurs non protéiques, en particulier des oestrogènes, des caroténoïdes et des cytokines.
